# EUROPEAN PATENT APPLICATION

(11) **EP 4 670 772 A1**
(43) Date of publication of application: **31.12.2025**
(21) Application number: 24774869.2
(22) Date of filing: 15.03.2024
(51) Int. Cl.: A61M 25/04, A61M 25/01, A61M 25/09, A61F 2/90

(54) **DISTAL STABILIZER**

(30) Priority: 17.03.2023 JP 2023042724; 08.06.2023 JP 2023094662
(71) Applicant: Bolt Medical Inc., Tokyo, 101-0021 (JP)
(72) Inventor: INUZUKA Naoki, Tokyo 103-0023 (JP)
(74) Representative: Appleyard Lees IP LLP
(86) International application number: PCT/JP2024/010280
(87) International publication number: WO 2024/195733

(57) **Abstract**

A distal stabilizer 1 for use in catheter delivery in a living body lumen is provided with: a linear delivery member; and a cylindrical part 2 connected to the linear delivery member and locked to the inner wall of the living body lumen. The cylindrical part 2 includes a body part 11 having a mesh pattern structure in which cells 20 having a shape surrounded by wire members are arranged along at least the major axis direction. At least one of the cells 20 forming the body part 11 has at least one open cell part 21 in which two wire members are connected at the top part 25. In at least one of the open cell parts 21, in a reduced diameter state, the two wire members extend along the major axis direction, and, in a natural state, the top part 25 protrudes along the circumferential direction orthogonal to the major axis direction.

## Description

### TECHNICAL FIELD

The present invention relates to, for example, a distal stabilizer designed for catheter delivery in a biological lumen.

### BACKGROUND ART

Certain treatments are administered by means of a catheter guided into a biological lumen, such as a patient's artery, so that a distal end of the catheter is placed in a vicinity of a target position. In this connection, the catheter is used to deliver a treatment device to the target position, or the catheter itself is used as a treatment device. For example, Patent Document 1 discloses an anchoring device (distal stabilizer) including a delivery wire and a cylindrical part joined to a distal end of the delivery wire. When the cylindrical part is released from a microcatheter and is allowed to expand, the cylindrical part is locked (anchored) to an inner wall of a biological lumen. Accordingly, it is possible to facilitate an operation of delivering a treatment catheter placed over the microcatheter to a vicinity of a target position. Furthermore, Patent Document 2 discloses a distal stabilizer in which a protruding free end of an open cell portion protrudes only toward a distal side. The distal stabilizer, which has the open cell portion, results in such characteristics including good anchoring force to an inner wall of a biological lumen and flexible conformity to a bend of the biological lumen.

### Citation List

### Patent Document

Patent Document 1: US Patent No. 968221
Patent Document 2: WO 2022/162965 A1

### DISCLOSURE OF THE INVENTION

### Problems to be Solved by the Invention

A treatment catheter that is placed over a microcatheter often has a large outer diameter and high rigidity. Accordingly, while being advanced, the treatment catheter frequently exerts a force that pulls the microcatheter with low rigidity and a delivery wire toward a proximal side. In particular, when a blood vessel that is a biological lumen includes a highly tortuous blood vessel, the treatment catheter may be easily caught at a bending portion or a branching portion, and, when a force is applied to allow the treatment catheter to pass through such a portion, a force that pulls the microcatheter and the delivery wire toward the proximal side is frequently exerted.

As the force that pulls the delivery wire toward the proximal side is applied, a position of the cylindrical part locked to an inner wall of the biological lumen may deviate toward the proximal side. A positional deviation of the cylindrical part occurs not only when the treatment catheter is delivered to a vicinity of the target position, but also when the cylindrical part locked to the inner wall of the biological lumen is resheathed into the catheter. If the position of the cylindrical part locked in the biological lumen is deviated toward the proximal side, it may be difficult to deliver a tip of the treatment catheter to a vicinity of the target position. A possible solution to reduce a positional deviation of the cylindrical part is to increase an expanding force (force for supporting a blood vessel) of the cylindrical part. However, increasing the expanding force of the cylindrical part may not only place greater stress on a blood vessel wall, but also reduce pushability of the cylindrical part due to an increase in sliding resistance between the microcatheter and the inner wall. A decrease in pushability of the cylindrical part also occurs when, for example, the cylindrical part is delivered toward a distal side in a highly tortuous blood vessel. As described above, an improvement in pushability is a design issue not only in a case of a cylindrical part having an increased expanding force, but also in a case of a cylindrical part having an ordinary expanding force. In the distal stabilizer of Patent Document 2 described above, in which the protruding free end of the open cell portion protrudes only toward the distal side, there is such a design issue that it is difficult to improve pushability of the cylindrical part.

In view of the design issue described above, an object of the present invention is to provide a distal stabilizer that has an open cell portion, that reduces a positional deviation of a cylindrical part locked in a biological lumen, and that further improves pushability of the cylindrical part.

### Means for Solving the Problems

First disclosure: A distal stabilizer designed for catheter delivery in a biological lumen includes: a linear delivery member; and a cylindrical part coupled to the linear delivery member and locked to an inner wall of the biological lumen. The cylindrical part includes a main body having a mesh-patterned structure formed of cells, each of which is enclosed by wire-shaped members. The cells are arranged in at least a major axis direction, and at least one cell among the cells forming the main body has at least one open cell portion in which two wire-shaped members are coupled to each other at a vertex. The at least one open cell portion includes the two wire-shaped members that extend in the major axis direction in a reduced diameter state and the vertex that protrudes in a circumferential direction orthogonal to the major axis direction in a natural state.

Second disclosure: The distal stabilizer described in the first disclosure, in which the open cell portion includes the vertex that protrudes in a direction at angles ranging from -30° to +30° inclusive with respect to the circumferential directions of the cylindrical part in the natural state.

Third disclosure: The distal stabilizer described in the first disclosure or the second disclosure, in which the open cell portion is provided to at least one cell among the cells, the at least one cell being positioned at a distal end in the major axis direction of the main body.

Fourth disclosure: The distal stabilizer described in the first disclosure or the second disclosure, in which the open cell portion is provided to at least one cell among the cells, the at least one cell being positioned at a proximal end in the major axis direction of the main body.

Fifth disclosure: The distal stabilizer described in the first disclosure or the second disclosure, in which the open cell portion is provided to at least one cell among the cells, the at least one cell being positioned between a distal end and a proximal end in the major axis direction of the main body.

Sixth disclosure: The distal stabilizer described in the first disclosure or the second disclosure, in which the open cell portion is also provided to an antenna portion coupling a proximal end of the main body to the linear delivery member.

Seventh disclosure: The distal stabilizer described in the first disclosure or the second disclosure, in which at least one cell among the cells, which has the open cell portion, includes an open cell portion in which some of the wire-shaped members do not extend substantially in the major axis direction in the reduced diameter state, but protrude in the circumferential direction orthogonal to the major axis direction in the natural state.

Eighth disclosure: The distal stabilizer described in the first disclosure or the second disclosure, in which at least one cell among the cells, which has the open cell portion, includes an open cell portion in which some of the wire-shaped member do not extend in the major axis direction in the reduced diameter state and the natural state.

Ninth disclosure: The distal stabilizer described in the first disclosure or the second disclosure, in which the cylindrical part has a structure in which the cells are arranged helically with respect to the major axis direction.

Tenth disclosure: The distal stabilizer described in the first disclosure or the second disclosure, in which the distal stabilizer is designed for an application in which the distal stabilizer is made to temporarily indwell in the biological lumen, and then collected out of a body.

Eleventh disclosure: A delivery method for a catheter system is provided. The catheter system includes: a distal stabilizer including a linear delivery member and a cylindrical part that extends from a distal end of the linear delivery member and that is lockable to an inner wall of a biological lumen; a small catheter that is able to accommodate the cylindrical part in a reduced diameter state together with the linear delivery member; and at least one large catheter that is placed over the linear delivery member or the small catheter. The delivery method for the catheter system includes: a first step for releasing the cylindrical part accommodated in the small catheter in the reduced diameter state from a distal end of the small catheter and locking the cylindrical part to the inner wall at a position more distal on a distal side than a target position in the biological lumen; a second step for pulling the small catheter toward a proximal side and allowing the linear delivery member to be exposed in the biological lumen; and a third step for allowing the large catheter to advance toward the distal side in a state in which the large catheter is placed over the linear delivery member and disposing the large catheter at the target position in the biological lumen. At the first step, between the target position at which the treatment catheter is to be disposed and the position at which the cylindrical part is to be locked, the biological lumen has one or more bent portions. And at least when the third step is started, in a state in which the linear delivery member is pulled toward the proximal side and an exposed part of the linear delivery member is allowed to be in contact with the one or more bent portions of the biological lumen, the large catheter is allowed to advance toward the distal side in a state in which the large catheter is placed over the linear delivery member.

Twelfth disclosure: The delivery method for the catheter system, described in the eleventh disclosure, in which, at the first step, the cylindrical part is locked to an inner wall of a biological lumen, a bend of which is larger among a plurality of biological lumens that branch at a branching portion that is present at a position more distal than the target position.

Thirteenth disclosure: The delivery method for the catheter system, described in the eleventh disclosure or the twelfth disclosure, in which, at the first step, the cylindrical part is locked at a position: more distal on the distal side than the target position in the biological lumen; and at which the exposed part of the linear delivery member straddles one or more bent portions or branching portions of the biological lumen.

Fourteenth disclosure: A delivery method for a catheter system is provided. The catheter system includes: a distal stabilizer including a linear delivery member and a cylindrical part that extends from a distal end of the linear delivery member and that is lockable to an inner wall of a biological lumen; a small catheter that is able to accommodate the cylindrical part in a reduced diameter state together with the linear delivery member; and at least one large catheter placed over the small catheter. The delivery method for the catheter system, includes: a first step for releasing the cylindrical part accommodated in the small catheter in the reduced diameter state from a distal end of the small catheter and locking the cylindrical part to the inner wall at a position more distal on a distal side than a target position in the biological lumen; a second step for pulling the small catheter toward a proximal side and allowing the linear delivery member to be exposed in the biological lumen; a third step for allowing the large catheter to advance toward the distal side in a state in which the large catheter is placed over the linear delivery member and disposing the large catheter at the target position in the biological lumen; a fourth step for reducing in diameter the cylindrical part, accommodating the cylindrical part in a collection catheter, and removing the cylindrical part out of the biological lumen; and a fifth step for inserting a treatment device into the large catheter, allowing the treatment device to advance toward the distal side, pushing the treatment device out of the large catheter at the target position, and delivering the treatment device to the target position. At the first step, between the target position at which the treatment catheter is to be disposed and the position at which the cylindrical part is to be locked, the biological lumen has one or more bent portions. And at least when the third step is started, in a state in which the linear delivery member is pulled toward the proximal side and an exposed part of the linear delivery member is allowed to be in contact with the one or more bent portions of the biological lumen, the large catheter is allowed to advance toward the distal side in a state in which the large catheter is placed over the linear delivery member.

Fifteenth disclosure: The delivery method for the catheter system, described in the fourteenth disclosure, in which, at the first step, the cylindrical part is locked to an inner wall of a biological lumen, a bend of which is larger among a plurality of biological lumens, which branches at a branching portion that is present at a position more distal than the target position.

Sixteenth disclosure: The delivery method for the catheter system, described in the fourteenth disclosure or the fifteenth disclosure, in which, at the first step, the cylindrical part is locked at a position: more distal on the distal side than the target position in the biological lumen; and at which the exposed part of the linear delivery member straddles one or more bent portions or branching portions of the biological lumen.

Seventeenth disclosure: A treatment method using a catheter system is provided. The catheter system includes: a distal stabilizer including a linear delivery member and a cylindrical part that extends from a distal end of the linear delivery member and that is lockable to an inner wall of a biological lumen; a small catheter that is able to accommodate the cylindrical part in a reduced diameter state together with the linear delivery member; and at least one large catheter placed over the small catheter. The treatment method using the catheter system includes: a first step for releasing the cylindrical part accommodated in the small catheter in the reduced diameter state from a distal end of the small catheter and locking the cylindrical part to the inner wall at a position more distal on a distal side than a target position in the biological lumen; a second step for pulling the small catheter toward a proximal side and allowing the linear delivery member to be exposed in the biological lumen; a third step for allowing the large catheter to advance toward the distal side in a state in which the large catheter is placed over the linear delivery member and disposing the large catheter at the target position in the biological lumen; a fourth step for reducing in diameter the cylindrical part, accommodating the cylindrical part in a collection catheter, and removing the cylindrical part out of the biological lumen; a fifth step for inserting a treatment device into the large catheter, allowing the treatment device to advance toward the distal side, pushing the treatment device out of the large catheter at the target position, and delivering the treatment device to the target position; and a sixth step for performing a treatment using the catheter system delivered to the target position. At the first step, between the target position at which the treatment catheter is to be disposed and the position at which the cylindrical part is to be locked, the biological lumen has one or more bent portions. And at least when the third step is started, in a state in which the linear delivery member is pulled toward the proximal side and an exposed part of the linear delivery member is allowed to be in contact with the one or more bent portions of the biological lumen, the large catheter is allowed to advance toward the distal side in a state in which the large catheter is placed over the linear delivery member.

Eighteenth disclosure: The treatment method using the catheter system, described in the seventeenth disclosure, in which, at the first step, the cylindrical part is locked to an inner wall of a biological lumen, a bend of which is larger among a plurality of biological lumens which branches at a branching portion that is present at a position more distal than the target position.

Nineteenth disclosure: The treatment method using the catheter system, described in the seventeenth disclosure or the eighteenth disclosure, in which, at the first step, the cylindrical part is locked at a position: more distal on the distal side than the target position in the biological lumen; and at which the exposed part of the linear delivery member straddles one or more bent portions or branching portions of the biological lumen.

In the eleventh disclosure, the fourteenth disclosure, and the seventeenth disclosure described above, the cylindrical part may be identical to or different from the cylindrical part of the first disclosure in mesh-patterned structure. For example, a protruding free end (described later) of the open cell portion of the cylindrical part may only protrude toward the proximal side, or an open cell portion in which a protruding free end protrudes toward the distal side and an open cell portion in which a protruding free end protrudes toward the proximal side may be mixed. Furthermore, the cylindrical part may include an open cell portion and a closed cell portion in a mixed manner or may include only a closed cell portion.

### Effects of the Invention

According to the present invention, it is possible to provide a distal stabilizer that has an open cell portion, that reduces a positional deviation of a cylindrical part locked in a biological lumen, and that further improves pushability of the cylindrical part.

### BRIEF DESCRIPTION OF THE DRAWINGS

FIG. 1 is a diagram illustrating a delivery system 10 including a distal stabilizer 1 according to an embodiment;
FIG. 2 is a diagram when a locking stent 2 according to a first configuration that is developed virtually onto a plane is viewed from inside;
FIG. 3A is a diagram when the locking stent 2 in a reduced diameter state that is developed virtually onto a plane is viewed from inside;
FIG. 3B is a diagram when an ordinary locking stent in a reduced diameter state that is developed virtually onto a plane is viewed from inside;
FIG. 4 is a conceptual diagram for describing directions in which vertexes 25 of open cell portions 21 protrude with respect to a circumferential direction RD;
FIG. 5 is a diagram when a locking stent 2 according to a second configuration that is developed virtually onto a plane is viewed from inside;
FIG. 6 is a schematic diagram illustrating a first example of a surgical procedure with a delivery system 10 including a distal stabilizer 1;
FIG. 7 is a schematic diagram illustrating the first example of the surgical procedure with the delivery system 10 including the distal stabilizer 1;
FIG. 8 is a schematic diagram illustrating the first example of the surgical procedure with the delivery system 10 including the distal stabilizer 1;
FIG. 9 is a schematic diagram illustrating the first example of the surgical procedure with the delivery system 10 including the distal stabilizer 1;
FIG. 10 is a schematic diagram illustrating the first example of the surgical procedure with the delivery system 10 including the distal stabilizer 1;
FIG. 11 is a schematic diagram illustrating the first example of the surgical procedure with the delivery system 10 including the distal stabilizer 1;
FIG. 12 is a schematic diagram illustrating the first example of the surgical procedure performed using the delivery system 10 including the distal stabilizer 1;
FIG. 13 is a schematic diagram illustrating the first example of the surgical procedure with the delivery system 10 including the distal stabilizer 1;
FIG. 14 is a schematic diagram illustrating the first example of the surgical procedure with the delivery system 10 including the distal stabilizer 1;
FIG. 15 is a schematic diagram illustrating a second example of a surgical procedure with a delivery system 10 including a distal stabilizer 1;
FIG. 16 is a schematic diagram illustrating the second example of the surgical procedure with the delivery system 10 including the distal stabilizer 1;
FIG. 17 is a schematic diagram illustrating the second example of the surgical procedure with the delivery system 10 including the distal stabilizer 1;
FIG. 18 is a schematic diagram illustrating the second example of the surgical procedure with the delivery system 10 including the distal stabilizer 1;
FIG. 19 is a schematic diagram illustrating the second example of the surgical procedure with the delivery system 10 including the distal stabilizer 1;
FIG. 20 is a schematic diagram illustrating the second example of the surgical procedure with the delivery system 10 including the distal stabilizer 1;
FIG. 21 is a schematic diagram illustrating the second example of the surgical procedure with the delivery system 10 including the distal stabilizer 1; and
FIG. 22 is a schematic diagram illustrating the second example of the surgical procedure with the delivery system 10 including the distal stabilizer 1.

### PREFERRED MODE FOR CARRYING OUT THE INVENTION

An embodiment of a distal stabilizer according to the present invention will be described below. Note that all of the drawings attached to the present specification are schematic diagrams or conceptual diagrams, and the shape, the scale, the vertical and horizontal dimensional ratio, and the like of each part are changed or exaggerated from actual ones in consideration of ease of understanding and the like. For example, illustrated catheters and other components appear to have a shorter dimension in a longitudinal direction and a larger dimension in a radial direction. In the present specification and the accompanying documents, terms specifying shapes, geometric conditions, and levels thereof, examples of which include "direction", etc., each encompass not only a strict meaning of the respective terms, but also an extent which can be regarded as approximately being in the direction by widely interpreting the respective terms. Accordingly, an "~ direction" may also appropriately be referred to as an "~ side".

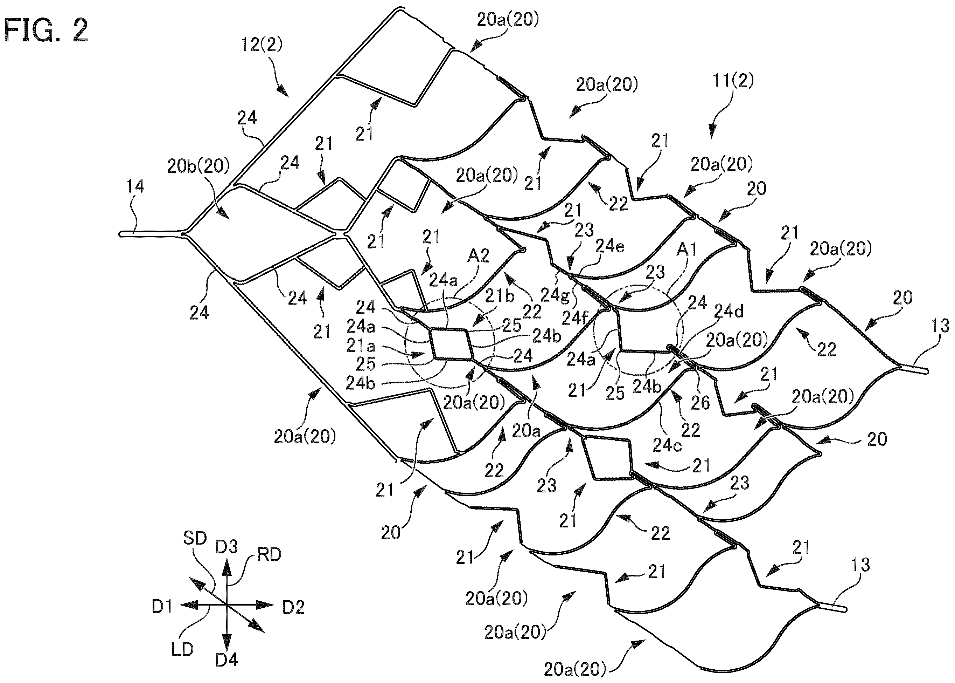

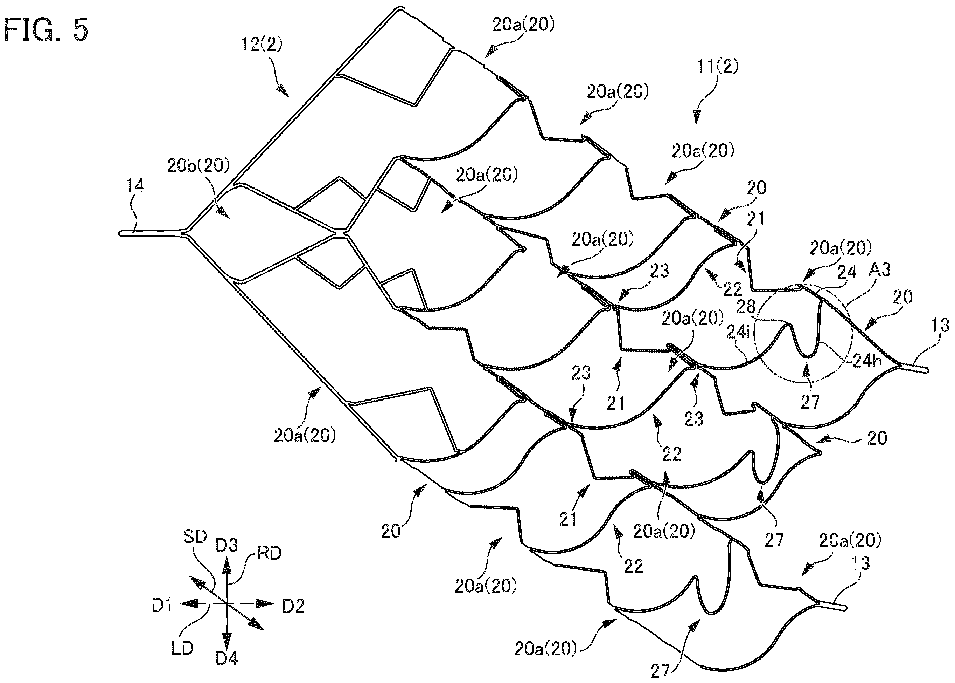

In the present specification, a major axis direction in a state in which a distal stabilizer 1 is extended linearly may also be referred to as "major axis direction LD". In a direction in which a locking stent 2 (described later) is inserted into a catheter, a proximal side close to a practitioner in the major axis direction LD is denoted by "D1", and a distal side away from the practitioner is denoted by "D2". Furthermore, a direction orthogonal to the major axis direction LD is referred to as "radial direction RD" or may also simply referred to as the "radial direction". In a state in which the locking stent 2 is developed virtually onto a plane (see FIG. 2), the radial direction RD corresponds to a circumferential direction of the locking stent 2. Accordingly, in a description of a state in which the locking stent 2 is developed virtually onto a plane, the "radial direction RD" may also appropriately be referred to as "circumferential direction RD". The circumferential direction RD includes one "circumferential direction D3" and another "circumferential direction D4" (see FIGs. 2 and 5).

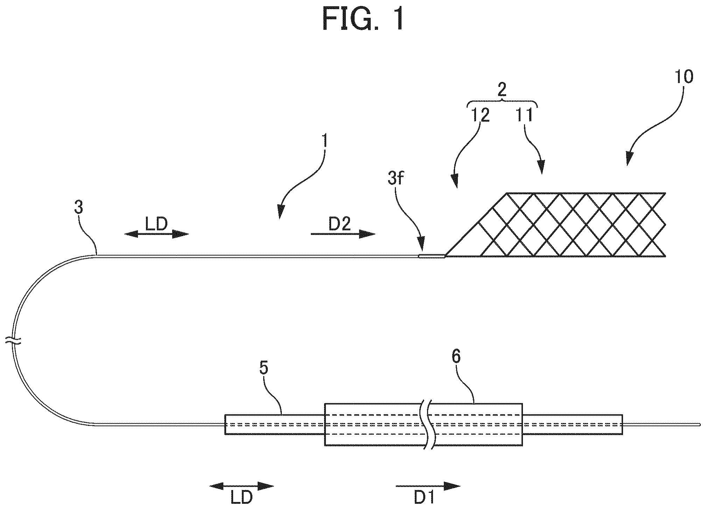

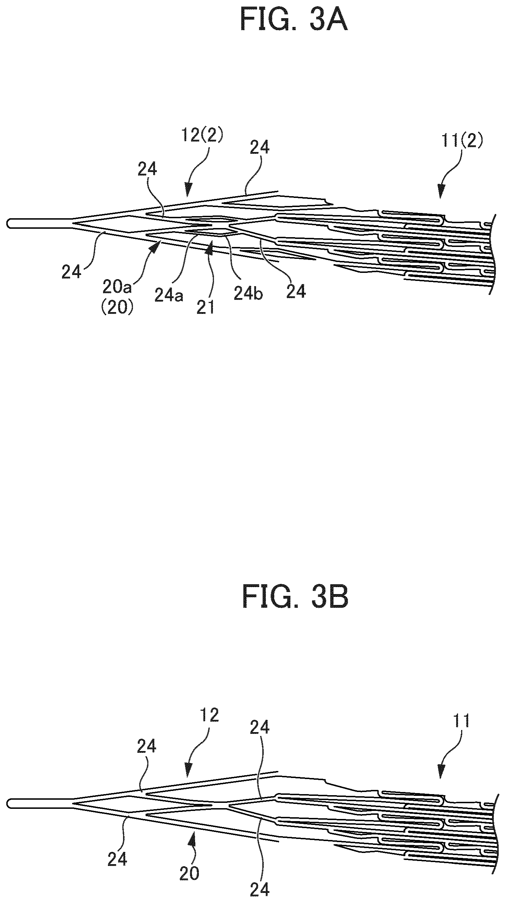

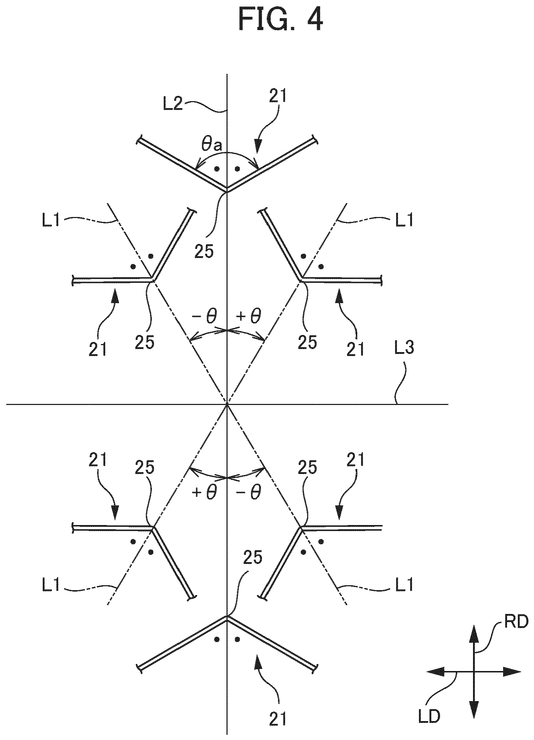

FIG. 1 is a diagram illustrating a delivery system 10 including a distal stabilizer 1 according to an embodiment. FIG. 2 is a diagram when a locking stent 2 according to a first configuration that is developed virtually onto a plane is viewed from inside. FIG. 3A is a diagram when the stent 2 in a reduced diameter state that is developed virtually onto a plane is viewed from inside. FIG. 3B is a diagram when an ordinary stent in a reduced diameter state that is developed virtually onto a plane is viewed from inside. FIG. 4 is a conceptual diagram for describing directions in which vertexes 25 of open cell portions 21 protrude with respect to the circumferential direction RD.

The delivery system 10 illustrated in FIG. 1 can be used to deliver a treatment device in a blood vessel that is a biological lumen, such as an intracranial blood vessel, examples of which include an anterior cerebral artery (ACA), an anterior communicating artery (Acom), a middle cerebral artery (MCA), and the like. Note that the blood vessel of the biological lumen in which the delivery system 10 is used is not limited to the examples described above. The blood vessel as the biological lumen may include, for example, a coronary artery or vein, a blood vessel (artery or vein) of an upper or lower limb, an organ, etc., in addition to the intracranial blood vessels. In the following description, the biological lumen is also referred to as a "blood vessel".

As illustrated in FIG. 1, the delivery system 10 includes the distal stabilizer 1 and a plurality of catheters including a first catheter 5 and a second catheter 6. The first catheter 5 is, for example, a catheter called a microcatheter. The second catheter 6 is placed over the first catheter 5. The second catheter 6 is a large-diameter catheter. A diameter of the first catheter 5 is set according to: a target position at which the second catheter 6 is to be delivered; and an inner diameter and degree of bend of a biological lumen as a path leading to the target position. It is not particularly limited, but the inner diameter of the first catheter 5 may be preferably 0.017 inches or less, and more preferably 0.0165 inches or less. One or more other catheters (not illustrated) that are placed over the second catheter 6 may be used as necessary. In general, using multiple catheters may make it possible to ultimately insert and advance a catheter having a large inner diameter into a biological lumen.

The distal stabilizer 1 is a device designed for catheter delivery in a biological lumen. The distal stabilizer 1 includes the locking stent (cylindrical part) 2 and a delivery wire (linear delivery member) 3.

The locking stent 2 is an anchoring device that is: inserted into the first catheter 5 while being in a reduced diameter state; and locked to an inner wall of a biological lumen upon expanding as a result of being released from the first catheter 5 in a blood vessel. The term "locked" to an inner wall of a biological lumen not only means that there is no deviation from a position resulting from expansion in the biological lumen, but also means that a positional deviation is allowed to an extent that there is substantially no influence on catheter delivery. The locking stent 2 is coupled to a distal end of the delivery wire 3 and extends therefrom. Note that the locking stent 2 is not limited to the example of being coupled to the distal end of the delivery wire 3, but may be coupled midway to the delivery wire 3. As illustrated in FIG. 1, the locking stent 2 includes a main body 11 and an antenna portion 12. The main body 11 is formed in a cylindrical shape and has a mesh-patterned structure described later. FIG. 1 depicts the configuration of the main body 11 in a simplified manner. The antenna portion 12 is where a portion on a proximal side D1 of the main body 11 converges to the delivery wire 3.

As illustrated in FIG. 2, distal markers 13 are provided to an end on a distal side D2 of the locking stent 2. A proximal marker 14 is provided to an end on the proximal side D1 of the locking stent 2. Each marker is made of an X-ray opaque material. The distal markers 13 serve as marks to check a position of an end on the distal side D2 of the locking stent 2. The proximal marker 14 serves as a mark to check a position of an end on the proximal side D1 of the locking stent 2.

The main body 11 has a structure in which a plurality of cells 20 are arranged in the major axis direction LD of the locking stent 2. In the present embodiment, the main body 11 is designed to have a mesh-patterned structure in which the plurality of cells 20 are arranged helically with respect to the major axis direction LD of the locking stent 2 to support an inner wall of a biological lumen when the main body is expanded in a blood vessel. As illustrated in FIG. 2, the plurality of cells 20 are arranged helically with respect to a cell arrangement direction (a cell arrangement direction as designed) SD that is inclined with respect to the major axis direction LD. In the present embodiment, as illustrated in FIG. 2, the cell arrangement direction SD is inclined upward in a rightward direction from the distal side D2 toward the proximal side D1 of the stent 2 (direction when viewed as the major axis direction LD is regarded as an upper-lower direction in the drawing). Note that the cell arrangement direction SD is not limited to the direction described above. The cell arrangement direction SD may be inclined upward in a leftward direction (inverted direction with respect to those illustrated in FIG. 2) from the distal side D2 toward the proximal side D1 of the locking stent 2. The term "in the cell arrangement direction SD" refers to being parallel or substantially parallel to the cell arrangement direction SD. The term "substantially parallel" refers to, for example, a range in which an angle between an arrangement direction of the cells 20 and the cell arrangement direction SD is about 1 to 15°. In the locking stent 2, for example, the angle at which the major axis direction LD intersects with the cell arrangement direction SD is less than 45°.

A cell 20, which is also referred to as an opening or compartment, represents a portion enclosed by wire-shaped struts (wire-shaped members) 24 that form the mesh pattern of the main body 11. In the locking stent 2 (main body 11) according to the present embodiment, as illustrated in FIG. 2, at least one cell among the cells 20 has open cell portions 21 and 22.

The at least one cell (hereinafter also referred to as a "cell 20a") among the cells 20, which has the open cell portions 21 and 22, may be provided in a desired region (the distal end, the proximal end, or between the distal end and the proximal end) in the major axis direction LD. In the main body 11, as illustrated in FIG. 2, a cell 20 having no open cell portion 21 and a cell 20a may be mixed with each other, or cells positioned in a region in the major axis direction LD may all be cells 20a. Furthermore, the numbers of cells 20a in respective regions may be identical to or different from each other. Moreover, a cell 20a may be provided in the major axis direction LD or the circumferential direction RD. In each of the directions, there may be one or more cells 20a.

An open cell portion 21 of a cell 20a is a portion in which ends of two substantially linear struts 24a and 24b are coupled to each other at a vertex 25 to form a substantially V-shape whole and no other struts are coupled to the vertex 25. The term, substantially linear, is not limited to being linear in a strict sense, but includes geometric states to be regarded linear roughly or generally. For cells 20a illustrated in regions A1 and A2 in FIG. 2, alphanumeric signs are applied to struts and vertexes forming open cell portions 21 (21a and 21b).

In the open cell portion 21a illustrated in the region A1 illustrated in FIG. 2, other ends of the two struts 24a and 24b are each coupled to a strut and/or a closed cell portion 23 (described later) in an identical column extending in the cell arrangement direction SD. On the other hand, the other ends of the struts 24a and 24b are not coupled to other components in the cell arrangement direction SD. Accordingly, the open cell portion 21 has a substantially V-shape.

Furthermore, in an open cell portion 21a illustrated in a region A2 in FIG. 2, struts to which other ends of struts 24a and 24b are coupled are coupled to other ends of struts 24a and 24b of an open cell portion 21b (21) of another adjacent cell 20a in the cell arrangement direction SD. In the region A2, a vertex 25 of the open cell portion 21a protrudes generally in the circumferential direction D4. A vertex 25 of the open cell portion 21b, on the other hand, protrudes generally in the circumferential direction D3. In the open cell portion 21a illustrated in the region A2, the other ends of the struts 24a and 24b are not coupled to other components in the cell arrangement direction SD. In the open cell portion 21b, similarly, the other ends of the struts 24a and 24b are not coupled to other components in the cell arrangement direction SD. Accordingly, the two open cell portions 21a and 21b of the two cells 20a adjacent to each other in the cell arrangement direction SD form a substantially diamond shape as a whole. The open cell portion 21a provided to the cell 20a may be one in number, as illustrated in the region A1, or may be coupled to the open cell portion 21b of another adjacent cell 20a each other, as illustrated in the region A2.

The shape of the open cell portion 21 is, for example, a substantially V-shape, U-shape, or Ω-shape. Although FIG. 2 illustrates the example in which one or more open cell portions 21 (21a and 21b) are provided to one cell 20a, the number and arrangement of the open cell portions 21 provided to one cell 20a are not limited to those in the example illustrated in FIG. 2.

A direction in which the vertex 25 protrudes in the open cell portion 21 of the cell 20a is not limited to a right-downward direction or a right-upward direction, as illustrated in FIG. 2, but may be a left-downward direction or a left-upward direction (when the major axis direction LD is regarded as an upper-lower direction in the drawing). Furthermore, it is possible to appropriately set a size of the open cell portion 21 (virtual area of a space enclosed by struts) in accordance with, for example, a size of the cell 20 provided with the open cell portion 21 or a rate of diameter reduction of the locking stent 2. The size of the open cell portion 21 may occupy, for example, a half area of a whole cell.

In the open cell portion 21 of the cell 20a, the two struts 24a and 24b extend in the major axis direction LD when the locking stent 2 is set in the reduced diameter state. The reduced diameter state refers to a state in which the locking stent 2 is reduced in diameter and is accommodated in the catheter (first catheter 5, for example). FIG. 3A illustrates that the two struts 24a and 24b of the open cell portion 21, which is provided to the antenna portion 12, extend in the major axis direction LD. In the antenna portion 12 in the reduced diameter state, which is illustrated in FIG. 3A, the number of the struts extending in the major axis direction is greater than the number of those in an ordinary locking stent illustrated in FIG. 3B.

Note that, as a structural requirement for allowing the struts 24a and 24b to extend easily in the major axis direction LD when the locking stent 2 is set in the reduced diameter state, it is possible to set an angle θa, at which the struts 24a and 24b, which are coupled to form the substantially V-shape, intersect with each other within a range of 100° to 160° inclusive, as illustrated in FIG. 4. Furthermore, in the open cell portion 21 of the cell 20a, it may be preferable that the other (opposite to a vertex side) ends of the struts 24a and 24b be each coupled to a strut and/or the closed cell portion 23 in the identical column extending in the cell arrangement direction SD. Satisfying such a structural requirement as described above in the open cell portion 21 of the cell 20a makes it possible to allow the struts 24a and 24b to extend easily in the major axis direction LD when the locking stent 2 is set in the reduced diameter state.

Note that, also in the open cell portion 21 provided to the main body 11, the two struts 24a and 24b (see FIG. 2) composing the open cell portion 21 form a shape (not illustrated) extending in the major axis direction LD when the locking stent 2 is set in the reduced diameter state. In the open cell portion 21 of the cell 20a, on the other hand, as will be described later, the vertex 25 protrudes in the circumferential direction RD orthogonal to the major axis direction LD when the locking stent 2 is set in a natural state. The natural state refers to a state in which the locking stent 2 is not reduced in diameter (no-load state).

As illustrated in FIG. 4, the vertex 25 of the open cell portion 21 protrudes in the circumferential direction RD orthogonal to the major axis direction LD when the locking stent 2 is set in the natural state. Specifically, the vertex 25 of the open cell portion 21 protrudes at angles ranging from -θ to +θ with respect to the circumferential direction RD of the main body 11 (locking stent 2). In FIG. 4, when a line bisecting the vertex 25 of the open cell portion 21 is designated as a line L1 indicating a direction in which the vertex 25 of the open cell portion 21 protrudes, a line parallel to the circumferential direction RD is designated as a line L2, and a line parallel to the major axis direction LD is designated as line L3, an angle (θ) at which the line L1 of the vertex 25 of the open cell portion 21 and the line L2 parallel to the circumferential direction RD intersect with each other ranges from 0° to +30° inclusive within a range of 0 (zero) to +θ inclusive and ranges from 0° to -30° inclusive within a range from 0 to -θ inclusive. More preferably, the angle (θ) ranges from 0° to +10° inclusive within a range from 0 (zero) to +θ inclusive and ranges from 0° to -10° inclusive within a range from 0 to -θ inclusive. When the line L1 of the vertex 25 of the open cell portion 21 and the line L2 coincide with each other, the angle (θ) is 0 (zero). The directions at an angle of "+θ" and "-θ" illustrated in FIG. 4 are applied for purposes of description, and the directions with the symbols "+" and "-" are not particularly limited. Furthermore, since FIG. 4 is a conceptual diagram, the shape, the size, and other factors of the open cell portion 21 are not limited to those in the illustrated example.

Note that, in the locking stent 2, the vertex 25 of the open cell portion 21 protrudes in the circumferential direction RD not only in the natural state, but also in a state in which the locking stent is released from the first catheter 5 in the blood vessel (hereinafter also referred to as an "expanded diameter state"). Then, even in the expanded diameter state, the vertex 25 of the open cell portion 21 of the locking stent 2 protrudes in a direction at angles ranging from -30° to +30° inclusive with respect to the circumferential direction RD of the main body 11.

Now back to FIG. 2, an open cell portion 22 of a cell 20 (cell 20a) is a portion in which ends of two struts 24c and 24d are coupled to each other at a vertex 26 and is a portion at which no other strut is coupled to the vertex 26. For the cells 20a positioned around a center in FIG. 2, alphanumeric signs are applied to struts and vertexes of the open cell portions 22. In the open cell portion 22, other ends of the two struts 24c and 24d are coupled to other struts or the closed cell portion 23 (described later). In the open cell portion 22, the two struts 24c and 24d do not extend substantially in the major axis direction LD in the reduced diameter state and the natural state. The term "do not extend substantially" means that, even if the two struts extend hypothetically in the major axis direction LD, the two struts do not extend to an extent that there is influence on pushability of the locking stent 2.

The shape of the open cell portion 22 is, for example, a substantially V-shape, U-shape, or Ω-shape. In the open cell portion 22 according to the present embodiment, the direction in which the vertex 26 protrudes generally toward the distal side D2 in the major axis direction LD, as illustrated in FIG. 2. Since the open cell portion 22 is less likely to be restrained by other struts, displacement and deformation in the radial direction RD are less likely to be restricted. Note that the direction in which the vertex 26 of the open cell portion 22 protrudes is not limited to that in the example illustrated in FIG. 2, and, for example, the vertex may extend in the cell arrangement direction SD.

The closed cell portion 23 of the cell 20 (cell 20a) is a portion in which ends on the proximal side D1 of two struts 24e and 24f are coupled to each other and another strut 24g is coupled to the coupled part. The closed cell portion 23 rarely changes in shape in the major axis direction LD and the circumferential direction RD when being in the reduced diameter state and the natural state, although such a change is observed in the open cell portion 21 described above. The shape of the closed cell portion 23 is, for example, a substantially V-shape, U-shape, or Ω-shape. It may be sufficient for the closed cell portion 23 to protrude generally toward the proximal side D1. As described above, the closed cell portion 23 protruding toward the proximal side D1, which is inhibited from outwardly warping by the strut 24g, is less likely to become an obstacle during being resheathed (accommodated) into the first catheter 5.

The antenna portion 12 is a portion that causes a part on the proximal side D1 of the main body 11 to converge to the delivery wire 3, and is coupled to the main body 11 in the major axis direction LD. The antenna portion 12 has a mesh-patterned structure as the main body 11 does. The antenna portion 12 is designed to cause a force supporting an inner wall of a biological lumen, which is generated during expansion in a blood vessel, to be relatively lower than that of the main body 11. The antenna portion 12 includes a plurality of cells 20, as the main body 11 does. The cells 20 forming the antenna portion 12 include a cell 20a that has open cell portions 21 and 22, and a cell 20 (hereinafter also referred to as a "cell 20b") that has no open cell portion. The cell 20b is, for example, a cell that is enclosed by four struts 24 and has no open cell portions 21 and 22. Note that the cells forming the antenna portion 12 are not limited to those in the example of the present embodiment. For example, the cells may include a cell including either an open cell portion 21 or 22. In addition, the cell may or may not include a closed cell portion 23.

A cell 20a includes two open cell portions 21 in the antenna portion 12 according to the present embodiment, as illustrated in FIG. 2. Alternatively, the cell 20a may include: one, three, or more open cell portions 21; or no open cell portion 21. Furthermore, a cell 20a that has an open cell portion 21 and a cell 20 that has no open cell portion 21 may be mixed. Moreover, the antenna portion 12 is not limited to the example that includes the cells 20a and 20b, and may include cells with a desired form, as long as functions serving as the antenna portion 12 are not impaired.

It is possible to produce the locking stent 2, for example, by performing laser processing on a tube made of a biocompatible material, particularly preferably, a superelastic alloy. In the case of production from a superelastic alloy tube, it may be preferable that a tube of about 2 mm to 3 mm is subjected in series to: laser processing; expansion to a desired diameter; and a shape memory treatment. The production of the main body 11 is not limited to laser processing, but the main body 11 may be produced by cutting processing or the like, or by braiding a wire-shaped metal into a tubular shape.

The locking stent 2 may be preferably made of a material having high rigidity and high biocompatibility. Examples of such a material include titanium, nickel, stainless steel, platinum, gold, silver, copper, iron, chromium, cobalt, aluminum, molybdenum, manganese, tantalum, tungsten, niobium, magnesium, calcium, and alloys containing these. Furthermore, as such a material, for example, a synthetic resin material of a polyolefin such as polyethylene (PE) or polypropylene (PP), polyamide, polyvinyl chloride, polyphenylene sulfide, polycarbonate, polyether, polymethyl methacrylate, or the like may be used. Moreover, as such a material, for example, a biodegradable resin (biodegradable polymer) such as polylactic acid (PLA), polyhydroxybutyrate (PHB), polyglycolic acid (PGA), or poly ε-caprolactone may be used.

Next, another configuration of a locking stent 2 will now be described herein. FIG. 5 is a diagram when a state in which the locking stent 2 according to a second configuration is developed virtually onto a plane is viewed from inside. The locking stent 2 according to the second configuration differs from the locking stent 2 according to the first configuration in that at least one cell among cells 20 of a main body 11 has open cell portions 21 and 27. Since other components in the locking stent 2 according to the second configuration are similar or identical to those of the locking stent 2 according to the first configuration, provision of some of the reference numerals is omitted, identical reference numerals are applied to equivalent components, and duplicated descriptions are omitted.

In the locking stent 2 according to the second configuration, as illustrated in FIG. 5, at least one cell 20a having the open cell portion 21 has an open cell portion 27. Although FIG. 5 illustrates an example in which cells 20a each having an open cell portion 27 are provided at a distal end in a major axis direction LD, the cell 20a having the open cell portion 27 may be provided at a desired position at a proximal end or between the distal end and the proximal end in the major axis direction LD. Furthermore, the cells 20a each having an open cell portion 27 may be provided in: the major axis direction LD; a circumferential direction RD; or helically in the major axis direction LD. In each of the directions, there may be one or more cells 20a each having an open cell portion 27.

In the locking stent 2 according to the second configuration, as illustrated in a region A3 of FIG. 5, the open cell portion 27 of the cell 20a is a portion in which ends of two struts 24h and 24i are coupled to each other at a vertex 28, and is a portion in which no other struts are coupled to the vertex 28. The strut 24h (part of a wire-shaped member) is formed, in a circumferential direction D4, for example, substantially into a U-shape or J-shape. Furthermore, the strut 24i (part of the wire-shaped member) is formed into a substantially curved shape. That is, in the open cell portion 27, neither of the two struts 24h and 24i are formed into a substantially linear shape. Accordingly, the open cell portion 27 is not wholly formed into a substantially V-shape, but has a shape having two bending portions, such as a substantially S-shape, N-shape, or Z-shape.

In the open cell portion 27, other ends of the two struts 24h and 24i are each coupled to a strut and/or a closed cell portion 23 of another adjacent cell 20 in a cell arrangement direction SD. That is, the open cell portion 27 differs from the open cell potion 21 in that the other ends of the struts 24h and 24i are not coupled to a strut and/or the closed cell portion 23 in a common column extending in the cell arrangement direction SD. Since the open cell portion 27 does not meet the structural requirement for allowing two struts to extend in the major axis direction LD when the locking stent 2 is set in a reduced diameter state as described above, the two struts do not extend in the major axis direction LD when the locking stent 2 is set in the reduced diameter state.

In the open cell portion 27, the shape of the strut 24h is not limited to a substantially U-shape, but may be, for example, a substantially V-shape or Ω-shape. In the open cell portion 27 of the cell 20a, the two struts 24h and 24i do not extend substantially in the major axis direction LD when the locking stent 2 is set in the reduced diameter state. Furthermore, in the open cell portion 27 of the cell 20a, the vertex 28 protrudes in the circumferential direction RD orthogonal to the major axis direction LD when the locking stent 2 is set in a natural state. A range of the direction in which the vertex 28 of the open cell portion 27 protrudes in the circumferential direction RD may be, for example, identical to a range of the direction in which the vertex 25 of the open cell portion 21 protrudes in the circumferential direction RD (see FIG. 4). Note that, as to the open cell portion 27 of the locking stent 2, the vertex 28 of the open cell portion 27 protrudes substantially in the circumferential direction RD not only in the natural state, but also in a released state from a first catheter 5 in a blood vessel, that is, in an expanded diameter state.

Next, a procedure that is a part of a surgical procedure of delivering a treatment device to a target position TP using a delivery system 10 according to the embodiment will now be described herein. Note that descriptions will be omitted for various operations that are performed as surgical procedures in addition to those described below. In the present embodiment, a biological lumen V is a blood vessel. In particular, a distal stabilizer 1 and the delivery system 10 according to the present embodiment are suitably used when the blood vessel includes a highly tortuous blood vessel. Furthermore, the distal stabilizer 1 and the delivery system 10 according to the present embodiment are preferably used when the target position TP is positioned in a region in which an inner diameter of the blood vessel is 7 mm or smaller, specifically, smaller than 2.5 mm (preferably, equal to or smaller than 2.0 mm, alternatively equal to or smaller than 1.5 mm). Specifically, the target position TP may be, for example, in a region corresponding to M2 or later (for example, M2, M3, or M4) of a middle cerebral artery (MCA), a region corresponding to A1 or A2 of an anterior cerebral artery (ACA), a region corresponding to P1 or later (for example, P1 or P2) of a posterior cerebral artery (PCA), or an internal carotid artery (ICA). However, the target position TP is not limited to those described above, and may be positioned in a region in which the inner diameter of the blood vessel ranges widely from 0.5 mm to 10 mm inclusive.

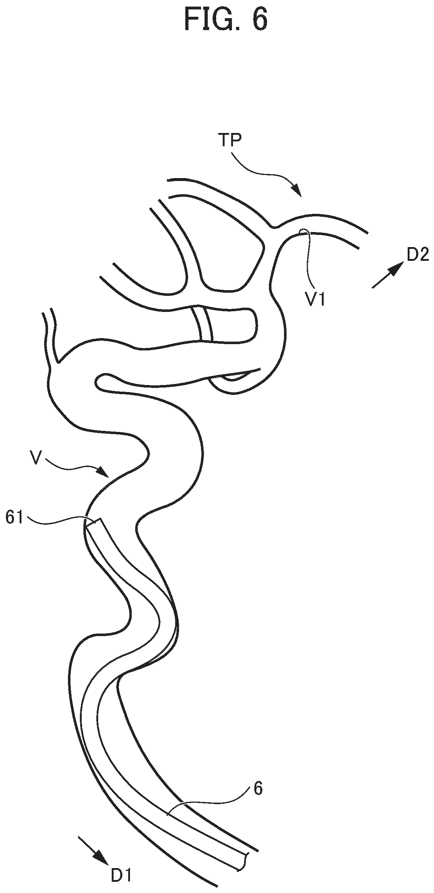

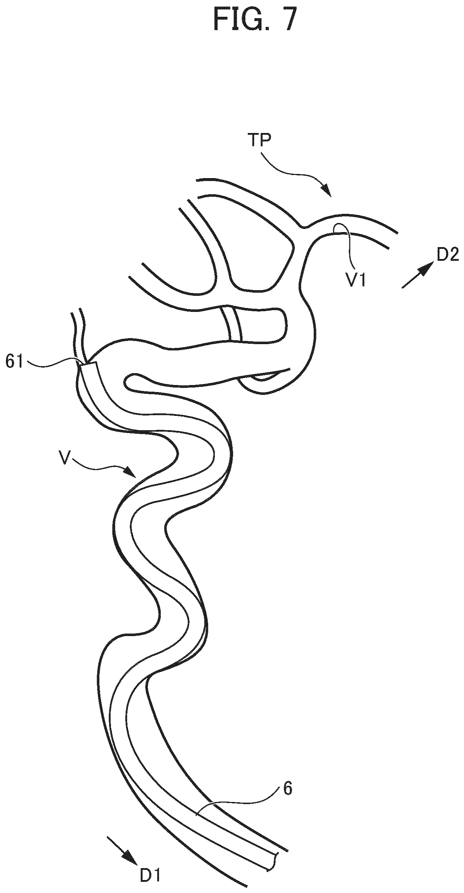

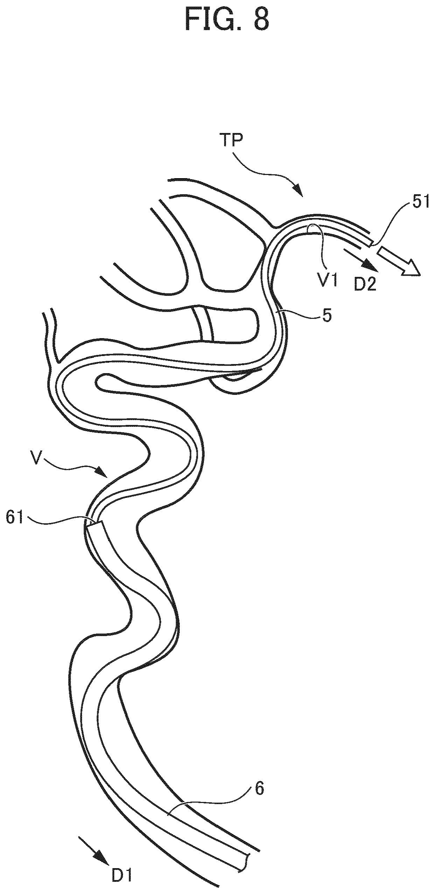

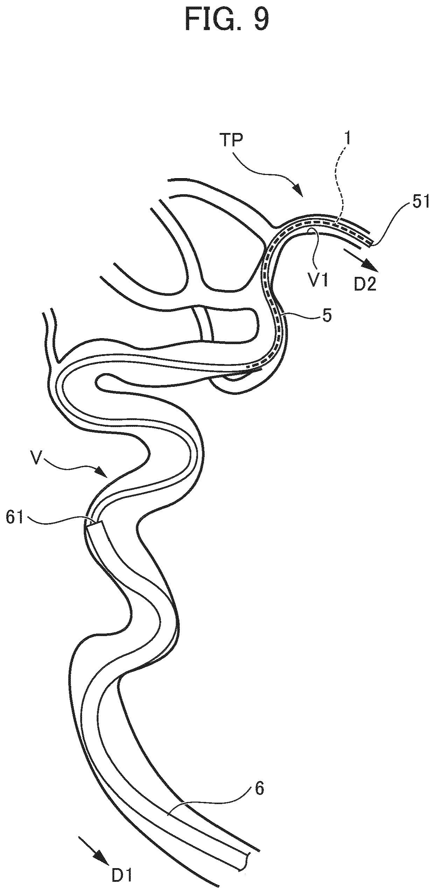

FIGs. 6 to 14 are schematic diagrams illustrating a first example of a surgical procedure performed using a delivery system 10 including a distal stabilizer 1. A second catheter 6 is first delivered, on a proximal side D1, into a biological lumen V of a patient. Typically, as illustrated in FIGs. 6 and 7, since a distal end 61 of the second catheter 6 is likely to be caught by a bent portion or a branching portion of the biological lumen V, it may be difficult to advance the second catheter 6 toward a distal side. A first catheter 5 is inserted into the second catheter 6, is delivered into the biological lumen V, and is pushed out of the distal end 61 of the second catheter 6, as illustrated in FIG. 8. Consequently, a distal end 51 of the first catheter 5 is delivered to a vicinity of a target position TP. Then, as illustrated in FIG. 9, the distal stabilizer 1 is inserted into the first catheter 5, and is delivered to the vicinity of the target position TP. At this time, a locking stent 2 (not illustrated) of the distal stabilizer 1 is accommodated in the first catheter 5 while being in a reduced diameter state. In the locking stent 2 in the reduced diameter state, as illustrated in FIG. 3A, two struts 24a and 24b of an open cell portion 21 form an extended shape in a major axis direction LD, similar to other struts 24. Accordingly, a propulsive force applied to a delivery wire 3 by a practitioner is easily transmitted in the major axis direction of the locking stent 2, improving pushability of the locking stent 2. Note that, for convenience, FIG. 9 depicts the distal stabilizer 1 (the locking stent 2 + the delivery wire 3) by a broken line, for only a portion on the distal side of the first catheter 5, although the distal stabilizer 1 is accommodated in an entirety of the first catheter 5.

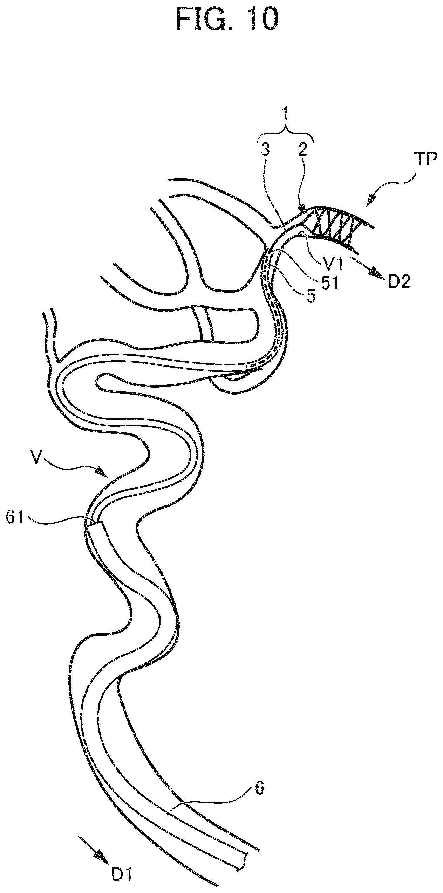

Next, as illustrated in FIG. 10, the locking stent 2 accommodated in the first catheter 5 while being in the reduced diameter state is released from the distal end 51 of the first catheter 5. Release of the locking stent 2 is performed, for example, by an operation of retracting the first catheter 5 toward the proximal side D1. The locking stent 2 released from the distal end 51 of the first catheter 5 expands in diameter due to its self-expanding force. Accordingly, the locking stent 2 pushes an inner wall V1 of the biological lumen V from inside toward outside to be locked to the inner wall V1 due to its frictional force.

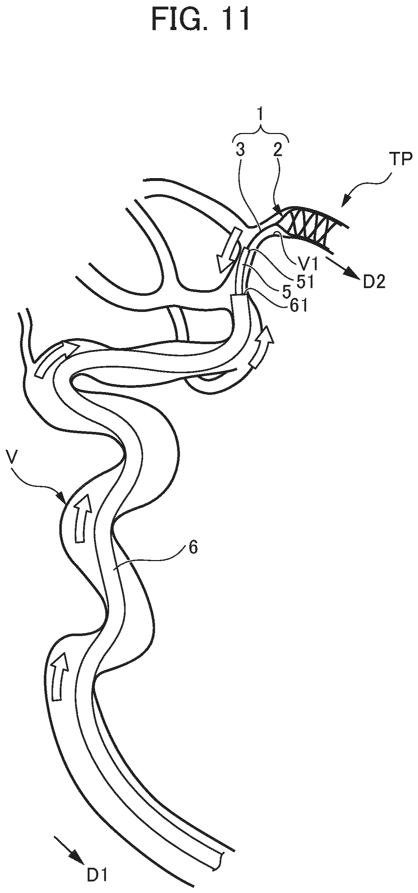

Then, as illustrated in FIG. 11, the second catheter 6 is placed over the first catheter 5, and the second catheter 6 is advanced toward a distal side D2 in the biological lumen V. Otherwise, after the locking stent 2 is locked to the inner wall V1 of the biological lumen V, the first catheter 5 may be removed, and the second catheter 6 is advanced along the delivery wire 3 toward the distal side D2 in the biological lumen V. The second catheter 6 has a large outer diameter, and its rigidity is high. Accordingly, while being advanced, the second catheter 6 frequently exerts a force that pulls the first catheter 5 and the delivery wire 3 with low rigidity toward the proximal side D1. As described above, when a force that pulls a delivery wire toward a proximal side is applied when using a conventional locking stent, a position of the conventional locking stent locked to the inner wall of a biological lumen may deviate. However, since, when the locking stent 2 according to the present embodiment is set in an expanded diameter state in the blood vessel, a vertex 25 of the open cell portion 21 protrudes in a circumferential direction RD to serve as a structural catch, it is possible to further improve an anchoring force through a synergistic effect with the frictional force due to the self-expanding force. Accordingly, it is possible to reduce a positional deviation of the locking stent 2, which may occur when a pulling force is frequently exerted. Furthermore, since the second catheter 6 is advanced in a state in which the delivery wire 3 is allowed to be in contact with the inner wall at a bent portion of the biological lumen in the surgical procedure of advancing the second catheter 6 along the delivery wire 3, a frictional force is generated at a portion that the inner wall and the delivery wire 3 are in contact with each other. Accordingly, it is possible to further improve an apparent anchoring force of the locking stent 2. In this description, the delivery wire 3 is pulled toward the proximal side D1 to allow the delivery wire 3 to come into contact with the inner wall of the biological lumen.

Note that, to perform such a surgical procedure as described above, it is necessary to make it possible to visually recognize a position and degree of bend of the delivery wire 3 in an X-ray transmission image. Specifically, there is a visually-recognizable part including an X-ray opaque part that is provided at a position on the distal side of the delivery wire 3 and that is bendable in an axial direction together with the delivery wire 3. The visually-recognizable part is provided in the axial direction from the distal end toward the proximal side of the linear delivery member. A length in the axial direction of the visually-recognizable part may range from 10 mm to 500 mm inclusive. It may be preferable that an X-ray opaque part is provided to occupy 10% or more of a surface region of the visually-recognizable part. The X-ray opaque part may include a tubular member provided on an outer peripheral surface of the delivery wire 3 or may include a wire-shaped member wrapped around the outer peripheral surface of the delivery wire 3. Furthermore, the X-ray opaque part may be provided in a continuous or discontinuous manner in the axial direction of the visually-recognizable part.

Example X-ray opaque materials include platinum, gold, tantalum, tungsten, iridium, platinum tungsten, and alloy materials thereof. Example alloy materials include polymer materials having an X-ray opaque property with the addition of an X-ray opaque filler.

Note that, when it is difficult to advance the second catheter 6, an operation of pulling the delivery wire 3 toward the proximal side D1 is sometimes performed, in a state in which the locking stent 2 is locked to the inner wall V1 of the biological lumen V. This pull operation can advance the second catheter 6 toward the distal side D2. Specifically, when the delivery wire 3 is pulled toward the proximal side D1 in a state in which the locking stent 2 is locked to the inner wall V1, a path of the delivery wire 3, which tends to be linear, shortens in the biological lumen V. At this time, since the position of a part on the proximal side D1 of the second catheter 6 does not change while this part is held directly or indirectly, the distal end 61 of the second catheter 6 instead advances in the biological lumen V, as illustrated in FIG. 11. Furthermore, when the distal end 61 of the second catheter 6 advances in the biological lumen V while the position of the part on the proximal side D1 of the second catheter 6 is held, the path of the second catheter 6 tends to be linear. This operation is particularly advantageous when the second catheter 6 needs to pass through a location which is difficult for the second catheter to pass through, such as a location in which the biological lumen V bends greatly and a location in which the inner diameter is small. With the distal stabilizer 1 according to the present embodiment, it is possible to reduce a positional deviation of the locking stent 2 locked to the inner wall V1 of the biological lumen V, even when such an operation as described above is performed.

Furthermore, when the locking stent 2 is to be locked at a position on the distal side of an aneurysm, and a treatment catheter having a large outer diameter is to be delivered to a vicinity of the aneurysm, the first catheter 5 inserted into the treatment catheter (second catheter 6) on the way forward sometimes strays into the aneurysm (not illustrated) together with the delivery wire 3. In such a case as described above, the path of the delivery wire 3 shortens in the biological lumen V by performing an operation of pulling the delivery wire 3 toward the proximal side D1 in a state in which the locking stent 2 is locked at a position on a distal side of the aneurysm. Accordingly, it is possible to restore the delivery wire 3 and the first catheter 5 from the aneurysm into the biological lumen V. As described above, it is possible for the distal stabilizer 1 according to the present embodiment to reduce a positional deviation of the locking stent 2 locked in the biological lumen V, when an operation of bringing a wire or a catheter having strayed into an aneurysm back into the biological lumen V is performed. Accordingly, it is possible to use the distal stabilizer 1 in an application in which the treatment catheter is delivered to a vicinity of a target position in a state in which the locking stent 2 is locked at a position more distal on the distal side than the aneurysm.

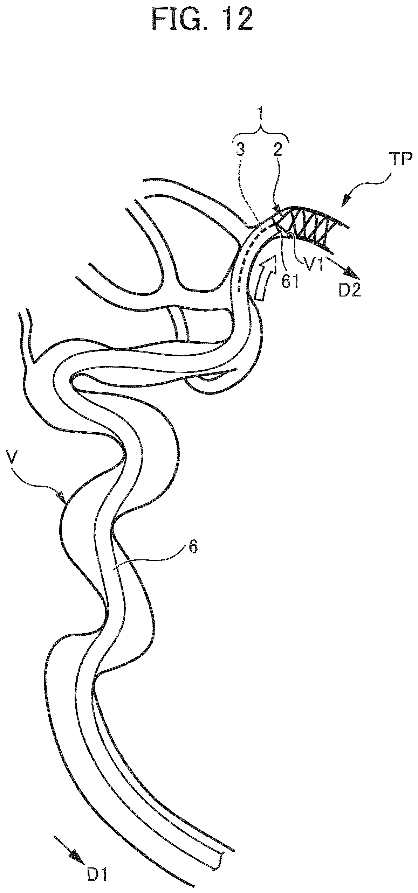

After the second catheter 6 is delivered as described above, as illustrated in FIG. 12, the distal stabilizer 1 is retracted toward the proximal side D1, and the stent 2 is resheathed

(accommodated) into the first catheter 5 (not illustrated). With the distal stabilizer 1 according to the present embodiment, it is possible to reduce a positional deviation of the locking stent 2 locked to the inner wall V1 of the biological lumen V during the resheathing, even when the locking stent 2 is to be resheathed. After the distal stabilizer 1 is resheathed into the first catheter 5, the first catheter 5 and the distal stabilizer 1 may be removed from the second catheter 6 on the proximal side D1. Otherwise, after the first catheter 5 is removed on the proximal side D1, the distal stabilizer 1 may be resheathed into the second catheter 6 and removed on the proximal side D1. Since, in the locking stent 2 according to the present embodiment, the direction in which the vertex 26 of the open cell portion 22 protrudes is generally in the major axis direction LD, resheathing into the first catheter 5 or the second catheter 6 is facilitated.

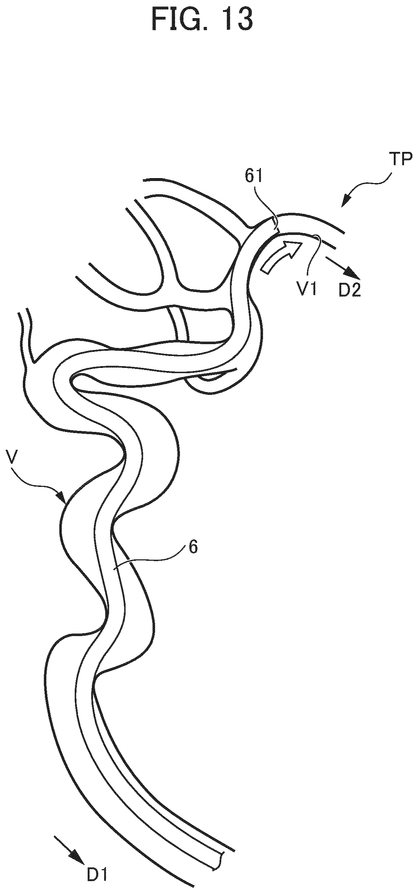

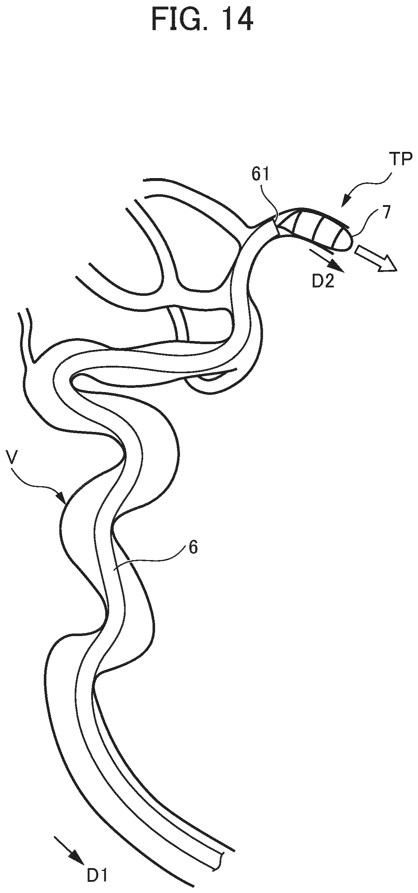

Next, as illustrated in FIG. 13, the second catheter 6 is disposed at the target position TP in the biological lumen V. Accordingly, as illustrated in FIG. 14, it is possible to easily deliver a treatment device 7 inserted into the second catheter 6 to the target position TP. Furthermore, the second catheter 6 or a third catheter itself may be used as a treatment device. When the second catheter 6 is used as a thrombus aspiration device, for example, an internal flow channel of the second catheter 6 is set to negative pressure, and a thrombus at the target position TP is aspirated and removed from the distal end 61. Note that removal of the distal stabilizer 1 is not limited to before delivering the treatment device 7, but may be after delivering the same. In the latter case, the second catheter 6 has a structure of two or more lumens including one lumen into which the distal stabilizer 1 and the first catheter 5 are to be inserted and another lumen into which the treatment device 7 is to be inserted.

The treatment device 7 is, for example, a thrombus aspiration device, a flow diverter, an embolic coil, an aneurysm embolization device, a thrombectomy device (such as a stent retriever), a stent for treating aneurysm, a stent for treating intracranial arterial stenosis, a balloon catheter, a shunt, or an embolic substance release means (such as a catheter having a lumen through which a liquid embolic substance passes). With the distal stabilizer 1 according to the present embodiment, it is possible to deliver each of the treatment devices described above in a transcatheter manner to a region in which the inner diameter of the blood vessel described above ranges from 0.5 mm to 10 mm inclusive, preferably equal to or below 7 mm, and specifically below 2.5 mm (preferably equal to or below 2.0 mm, or equal to or below 1.5 mm). Accordingly, it is possible to achieve a treatment or an improvement for a decease or disorder (such as a vascular occlusion or a blood vessel knob) with less stress on a patient in these regions.

Note that the treatment device 7 delivered to the target position TP may be used in a biological lumen (such as a thrombus aspiration device, a flow diverter, an aneurysm embolization device, a thrombectomy device, a stent for treating aneurysm, a stent for treating intracranial arterial stenosis, a balloon catheter, or an embolic substance release means), or may be used while protruding outside the biological lumen (such as a shunt). The distal stabilizer 1 according to the present embodiment is also applied to a treatment in which the delivery system 10 is used to deliver the treatment device 7 to the target position TP in the biological lumen V, and a treatment is performed at the target position TP in the biological lumen V or after protruding the treatment device 7 out of the biological lumen V from the target position TP.

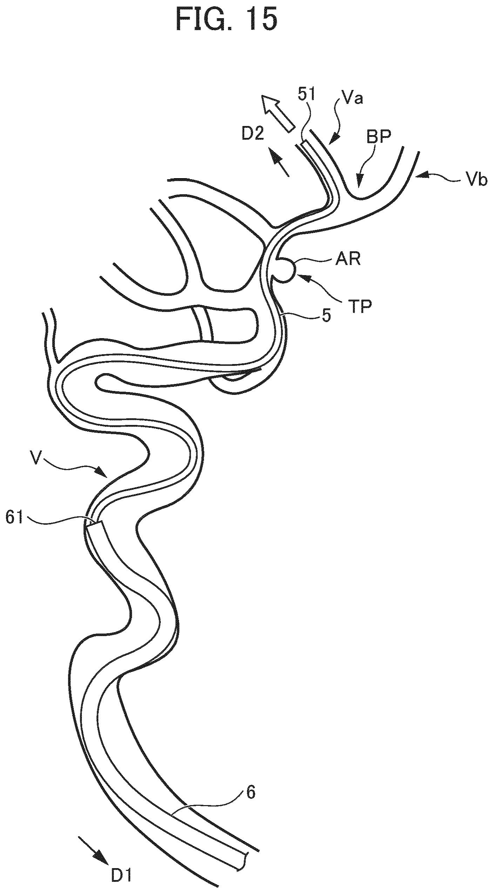

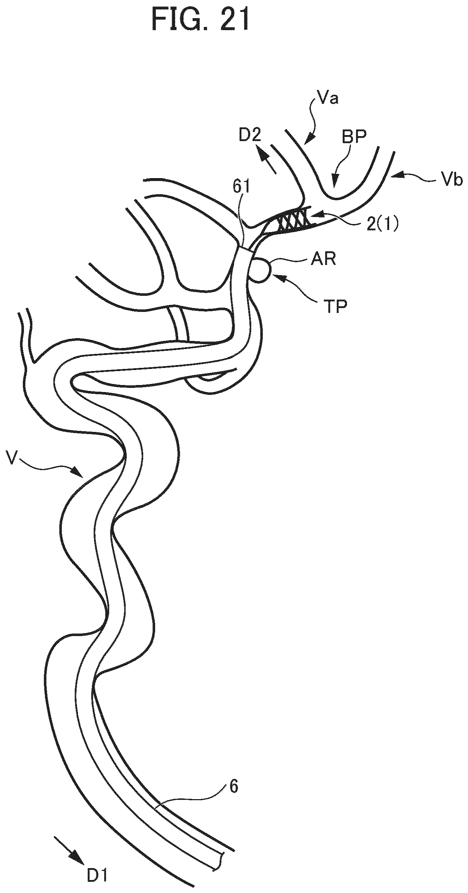

Next, a second example of a surgical procedure performed using a delivery system 10 including a distal stabilizer 1 will now be described herein with reference to FIGs. 15 to 22. In the second example of the surgical procedure, stages of the procedure before a first catheter 5 is delivered to a target position TP are substantially identical to those in the first example of the surgical procedure. Accordingly, in the second example of the surgical procedure, descriptions start from a stage in which the first catheter 5 is delivered to the target position TP, and the descriptions for the previous stages of the procedure are omitted. FIGs. 15 to 22 are schematic diagrams illustrating the second example of the surgical procedure performed using the delivery system 10 including the distal stabilizer 1.

In the second example of the surgical procedure, descriptions will be given here of an example in which an aneurysm that has formed at the target position TP in a biological lumen V is treated. Accordingly, in FIGs. 15 to 22, the target position TP and the shape of a blood vessel on a distal side, for example, differ from those in the first example of the surgical procedure. In the present example, the aneurysm at the target position TP is, for example, an aneurysm that has formed at a distal position of a tortuous blood vessel having a small diameter, such as an intracranial blood vessel, examples of which include an anterior cerebral artery (ACA), an anterior communicating artery (Acom), a middle cerebral artery (MCA), and the like. On the distal side of an internal carotid artery (ICA), many tortuous blood vessels are present. Accordingly, as illustrated in the second example of the surgical procedure, when an aneurysm formed in the middle cerebral artery (MCA) that is positioned at a position more distal on the distal side than the internal carotid artery is to be treated, it is important that a delivery wire 3 come into contact with many bent portions on the distal side of the internal carotid artery.

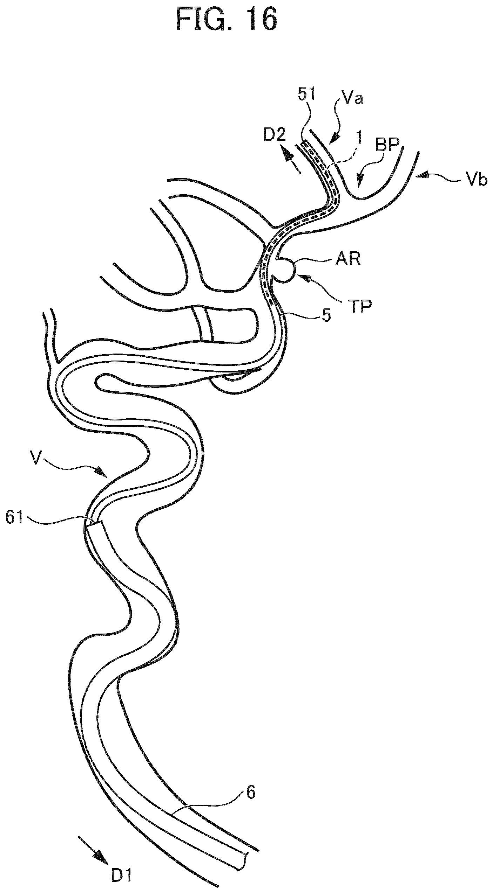

After a first catheter 5 is inserted into a second catheter 6 and delivered into the biological lumen V, and the first catheter 5 is pushed out of a distal end 61 of the second catheter 6, as illustrated in FIG. 15, a distal end 51 of the first catheter 5 is delivered to a position on the distal side of the target position TP. In the second example of the surgical procedure, the lumen is branched at a branching portion BP on the distal side of the target position TP into two biological lumens Va and Vb. FIG. 15 illustrates an example in which the distal end 51 of the first catheter 5 is delivered into the biological lumen Va that bends more than the biological lumen Vb. The term "bends more" means bending is relatively greatest, compared with other biological lumens among a plurality of branched biological lumens. Then, as illustrated in FIG. 16, the distal stabilizer 1 is inserted into the first catheter 5, and is delivered to a position on the distal side of the target position TP (biological lumen Va).

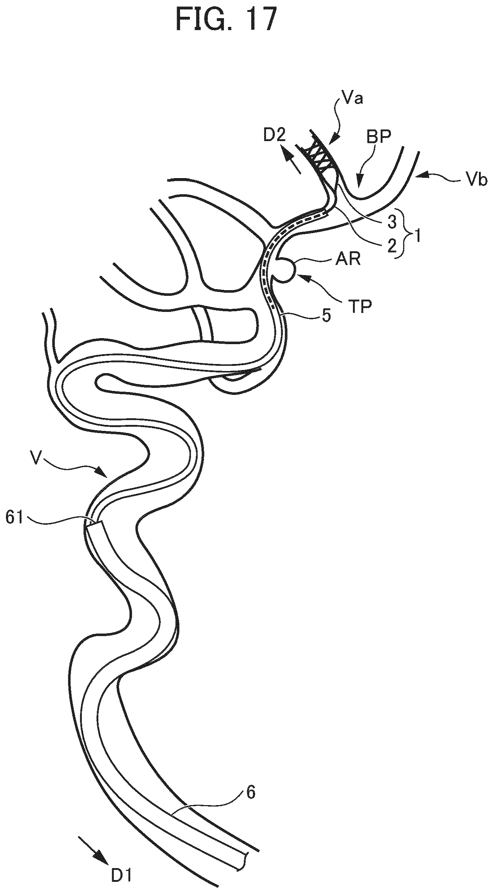

Next, as illustrated in FIG. 17, a locking stent 2 accommodated in the first catheter 5 while being in a reduced diameter state is released from the distal end 51 of the first catheter 5. The locking stent 2 released from the distal end 51 of the first catheter 5 expands in diameter due to its self-expanding force. Accordingly, the locking stent 2 is locked to an inner wall of the biological lumen Va that bends more at the branching portion BP (first step). Furthermore, the locking stent 2 is locked at a position where one or more bent portions are present between the target position TP and a position at which the locking stent 2 is locked. In the present example, the locking stent 2 is locked at a position where two bent portions of the biological lumen V are present between the target position TP and the position at which the locking stent 2 is locked. Between the target position TP and the position at which the locking stent 2 is locked, a maximum allowable number of bent portions is, for example, three when the diameter of a blood vessel at each of the bent portions ranges from 1.0 mm to 3.0 mm inclusive in a case of a middle cerebral artery. Furthermore, the number is four when the diameter of a blood vessel at each of the bent portions ranges from 1.0 mm to 3.0 mm inclusive in a case of an anterior cerebral artery.

As described above, by locking the locking stent 2 at a position more distal on the distal side than a position at which the second catheter 6 is to be delivered as much as possible, it is possible to further extend an exposed part of the delivery wire 3 in an area more distal on the distal side than the target position TP. Accordingly, since it is possible to secure a longer contact range between the delivery wire 3 and each of the bent portions of the biological lumen, it is possible to further improve the anchoring force of the locking stent 2. Note that, although it depends on a portion of a biological lumen, the exposed part of the delivery wire 3 is desirably equal to or longer than 10 mm and preferably equal to or longer than 20 mm. When the exposed part is equal to or longer than 50 mm, it is possible to allow the exposed part to come into contact with each bent portion in most biological lumens. Setting the exposed part of the delivery wire 3 to a length equal to or longer than 100 mm makes it possible to allow the exposed part to come into contact with a bent portion at any desired portion in a biological lumen. An upper limit of an appropriate length of the exposed part of the delivery wire 30 is, for example, approximately 70 mm in a case of the middle cerebral artery and approximately 50 mm in a case of the anterior cerebral artery.

Furthermore, the locking stent 2 is locked at a position on the distal side of the target position TP, at which the exposed part of the delivery wire 3 straddles one or more bent portions or one or more branching portions of the biological lumen V. In the present example, the locking stent 2 is locked at a position on the distal side of the target position TP, at which the exposed part of the delivery wire 3 straddles one bent portion and one branching portion of the biological lumen V, respectively. Note that the present invention is not limited to the present example, and the locking stent 2 may be locked at a position on the distal side of the target position TP, at which the exposed part of the delivery wire 3 straddles one or more bent portions of the biological lumen V or may be locked at a position at which the exposed part of the delivery wire 3 straddles one or more branching portions of the biological lumen V. Furthermore, the exposed part of the delivery wire 3 may straddle up to three branching portions of the biological lumen V, for example, when a diameter of a blood vessel at each of the bent portions ranges from 1.0 mm to 3.0 mm inclusive in a case of a middle cerebral artery. Furthermore, the number is four when the diameter of the blood vessel at each of the bent portions ranges from 1.0 mm to 3.0 mm inclusive in a case of an anterior cerebral artery.

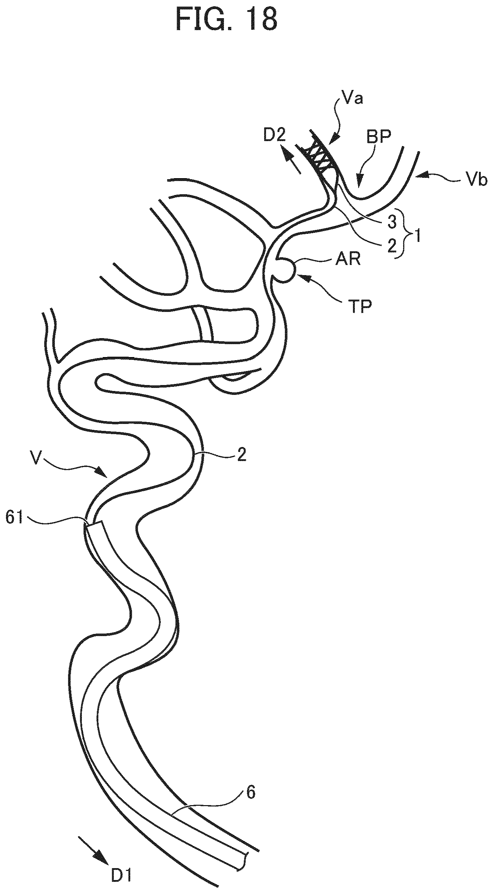

Next, as illustrated in FIG. 18, the first catheter 5 (not illustrated) is pulled toward a proximal side D1 and removed, and the delivery wire 3 is allowed to be exposed in the biological lumen V (second step). Alternatively, the first catheter 5 may not be removed from the second catheter 6, but may be retracted to a position in a vicinity of the distal end 61 of the second catheter 6. As will be described later, as long as it is possible to allow the exposed part of the delivery wire 3 to come into contact with one or more bent portions of the biological lumen V, the first catheter 5 may be removed or may not be removed.

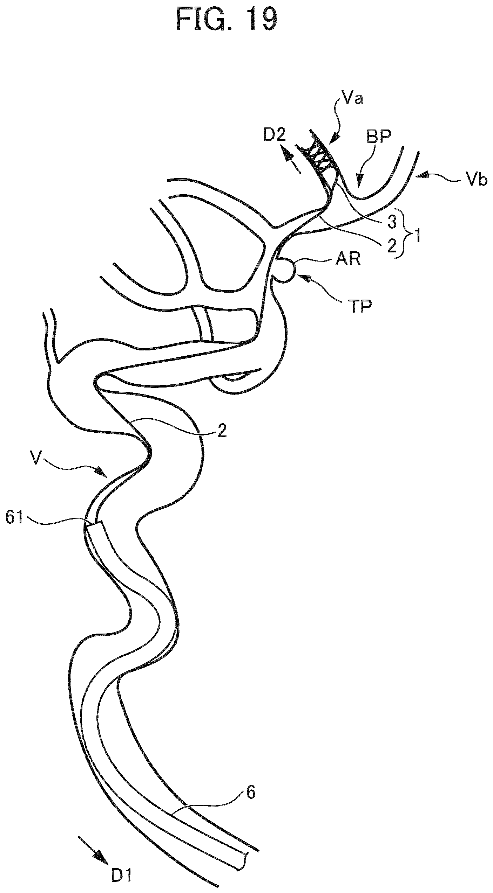

Then, as illustrated in FIG. 19, the delivery wire 3 is pulled toward the proximal side D1, and is allowed to come into contact with bent portions in the biological lumen V. In the present example, as the delivery wire 3 is pulled toward the proximal side D1, the exposed part of the delivery wire 3 comes into contact with three bent portions in the biological lumen V. Note that it is sufficient that the exposed part of the delivery wire 3 be in contact with one or more bent portions in the biological lumen V.

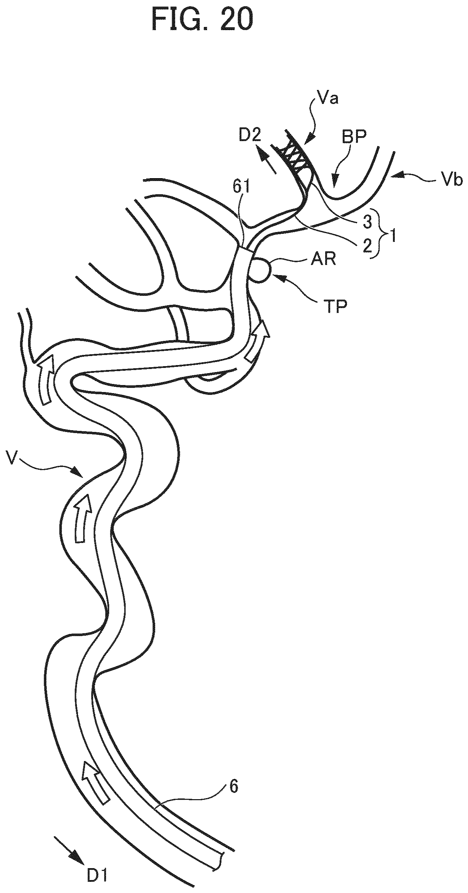

Next, as illustrated in FIG. 20, the second catheter 6 is advanced toward a distal side D2 while being placed over the delivery wire 3, and is disposed at the target position TP (third step). At least when the third step is started, and the second catheter 6 is advanced toward the distal side D2 while being placed over the delivery wire 3, in a state in which the exposed part of the delivery wire 3 is in contact with the one or more bent portions of the biological lumen V. The locking stent 2 is firmly locked to the inner wall on the distal side D2 of the target position TP. Furthermore, as will be described later, it is possible to advance the second catheter 6 toward the distal side D2 while the delivery wire 3 is in contact with the one or more bent portions in the biological lumen V. Accordingly, it is possible to further increase the frictional force exerted by an entirety of the distal stabilizer 1 against the biological lumen V. Therefore, it is possible to further securely retain the entirety of the distal stabilizer 1 in the biological lumen V against a force of pulling the delivery wire 3 toward the proximal side, which is generated when the second catheter 6 is advanced.

Furthermore, with the distal stabilizer 1 according to the present embodiment, it is possible to reduce the self-expanding force of the locking stent 2 while the frictional force exerted by the entirety of the distal stabilizer 1 against the biological lumen V is not reduced so much. Accordingly, it is possible to further reduce the stress on the blood vessel wall.

After the distal end 61 of the second catheter 6 reaches the target position TP in the biological lumen V, the second catheter 6 is further delivered toward the distal side D2, and the locking stent 2 is resheathed into the second catheter 6 (fourth step).

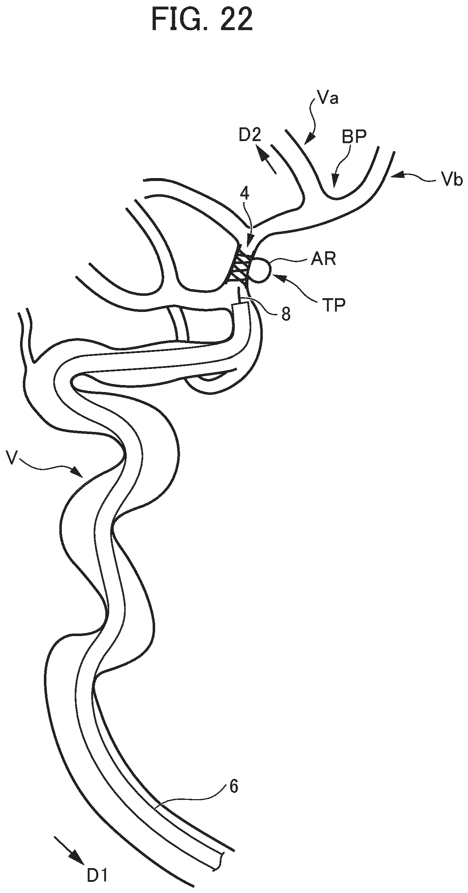

Next, although not illustrated, an indwelling stent 4 in the reduced diameter state is inserted into the second catheter 6 from the proximal side D1. The indwelling stent 4 is a stent for treating aneurysm (treatment device). Then, as illustrated in FIG. 22, the indwelling stent 4 is delivered toward the distal side D2 via a pusher wire 8, pushed out of the second catheter 6 at the target position TP, and delivered to the target position TP (fifth step). The purpose of making the indwelling stent 4 indwell at the target position TP is to reduce blood flowing into an aneurysm AR present at the target position TP or to retain an embolic coil indwelling in the aneurysm AR. After the indwelling stent 4 is made to indwell at the target position TP, an embolic coil (not illustrated) is delivered toward the distal side D2 through a microcatheter (not illustrated) inserted in the second catheter 6, whereby it is possible to allow the embolic coil to pass through the meshes of the indwelling stent 4 to indwell in the aneurysm AR (sixth step). Note that, as the indwelling stent 4 expands by itself, the pusher wire 8 pinched by the indwelling stent 4 comes off. Accordingly, it is possible to collect the pusher wire 8 while the indwelling stent 4 is left behind in the aneurysm part.

In the second example of the surgical procedure, in which the second catheter 6 is advanced along the delivery wire 3, it is possible to advance the second catheter 6 toward the distal side D2 in a state in which the delivery wire 3 is in contact with the inner wall of the one or more bent portions in the biological lumen V. That is, as illustrated in FIG. 19, when the second catheter 6 is to be advanced toward the distal side D2, the delivery wire 3 comes in contact with the one or more bent portions at one or more positions more distal on the distal side D2 than the target position TP. Furthermore, the delivery wire 3 comes in contact with the one or more bent portions at the one or more positions more distal on the proximal side D1 than the target position TP. Accordingly, the frictional force resulting from the contact between the delivery wire 3 and the inner wall of the biological lumen V further increases. As a result, it is possible to further improve the frictional force exerted by the distal stabilizer 1 against the biological lumen V. Therefore, with the distal stabilizer 1 according to the present embodiment, it is possible to further securely retain the distal stabilizer 1 at a locking position against a force of pulling the delivery wire 3 toward the proximal side, which is generated when the second catheter 6 is advanced.

The locking stent 2 is locked to the inner wall of the biological lumen Va that bends greatly at the branching portion BP in the biological lumen V. Accordingly, the frictional force resulting from the contact between the delivery wire 3 and the inner wall of the biological lumen Va further increases. As a result, it is possible to further improve the anchoring force of the distal stabilizer 1. Furthermore, the more the second catheter 6 is advanced toward the distal side D2, the more the exposed part of the delivery wire 3 is inserted into the second catheter 6. Accordingly, a number of locations at which bent portions and the delivery wire 3 are in contact with each other decreases as the second catheter 6 is advanced toward the distal side D2. However, in the second example of the surgical procedure, the contact between the delivery wire 3 and the one or more bent portions is maintained at positions more distal on the distal side D2 than the target position TP. According to this configuration, since it is possible to keep the anchoring force of the locking stent 2 even when the second catheter 6 is advanced toward the distal side D2, it is possible to stably advance the second catheter 6 until the distal end 61 of the second catheter 6 is disposed at the target position TP in the biological lumen V.

The distal stabilizer 1 according to the present embodiment described above exerts the following effects, for example.

In the open cell portion 21 provided to the cell 20a, in the locking stent 2 according to the first configuration, the two struts extend in the major axis direction LD when the locking stent 2 is set in the reduced diameter state. Accordingly, the propulsive force applied to the delivery wire 3 by a practitioner is easily transmitted in the major axis direction of the locking stent 2. As a result, it is possible to further improve pushability of the locking stent 2. Furthermore, in the main body 11 of the locking stent 2, the open cell portion 21 provided to the cell 20a includes the vertex 25 that protrudes in the circumferential direction RD orthogonal to the major axis direction LD when the locking stent 2 is set in the expanded diameter state. Accordingly, since the vertex 25 of the open cell portion 21 of the locking stent 2 serves as a structural catch with respect to the inner wall in the blood vessel, it is possible to further improve the anchoring force through a synergistic effect with the frictional force due to the self-expanding force.

In the locking stent 2 according to the first configuration, the vertex 25 of the open cell portion 21 provided to the cell 20a protrudes in a direction at angles ranging from -30° to +30° inclusive with respect to the circumferential direction RD of the locking stent 2 when the locking stent 2 is set in the expanded diameter state. According to this configuration, it is possible to allow the vertex 25 of the open cell portion 21 to protrude in a direction in which it is possible to achieve a sufficient structural catch in the expanded diameter state.

In the locking stent 2 according to the first configuration, the open cell portion 21 is provided to at least one cell positioned at the distal end in the major axis direction LD of the main body 11. Accordingly, it is possible to maintain the anchoring force of the locking stent 2 immediately before the locking stent 2 is resheathed into the catheter (first catheter 5).

In the locking stent 2 according to the first configuration, the open cell portion 21 is provided to at least one cell positioned at the proximal end in the major axis direction LD of the main body 11. Accordingly, it is possible to improve pushability at the proximal end of the main body 11, in which the density of the cells is likely to be sparser.

In the locking stent 2 according to the first configuration, the open cell portion 21 is provided to at least one cell positioned between the distal end and the proximal end in the major axis direction LD of the main body 11. According to this configuration, since it is possible to reduce struts in number in a region in which the cells are disposed denser and resistance generated during resheathing is likely to increase in the main body 11 and it is possible to relatively reduce an amount of metal in the region compared to that in other regions, it is possible to reduce the resistance generated during resheathing.

In the locking stent 2 according to the first configuration, the open cell portion 21 is also provided to the antenna portion 12. According to this configuration, the propulsive force applied to the delivery wire 3 by a practitioner is easily transmitted to the main body 11 in the antenna portion 12 in which the density of cells may be sparse. Accordingly, it is possible to further improve pushability of the locking stent 2.

In the locking stent 2 according to the first configuration, at least one cell having the open cell portion 21 has the open cell portion 22 in which the two struts do not extend substantially in the major axis direction LD in the reduced diameter state and the natural state. Allowing the vertex 26 of the open cell portion 22 to protrude toward the distal side D2 in the major axis direction LD makes resheathing into the first catheter 5 and the second catheter 6 easier.

In the locking stent 2 according to the second configuration, at least one cell 20a having the open cell portion 21 has the open cell portion 27. The open cell portion 27 includes the strut 24h (part of the wire-shaped member) that protrudes in the circumferential direction RD in the expanded diameter state. According to this configuration, since the vertex 28 of the open cell portion 27 serves as a structural catch with respect to the inner wall of the blood vessel in the expanded diameter state, it is possible to further improve the anchoring force through a synergistic effect with the frictional force resulting from the self-expanding force. In particular, disposing the cell having the open cell portion 27 on the distal side makes it possible to exert an anchoring force of the locking stent 2 immediately before the locking stent 2 is resheathed into the catheter (first catheter 5).

The locking stents according to the first and second configurations includes the main body 11 that has the mesh-patterned structure in which the plurality of cells 20 are arranged helically with respect to the major axis direction LD. Accordingly, since the distal stabilizer 1 having the locking stent has high flexibility, the distal stabilizer 1 follows easily the bends of the biological lumen V. As described above, since the distal stabilizer 1 easily follows the bends of the biological lumen V, stress is less likely to concentrate on opposite ends of the locking stent in the major axis direction LD. Accordingly this configuration provides an open cell structure that reduces stress on a blood vessel wall, since the blood vessel to which the locking stent is locked is less likely to straighten out.

It should be noted that the present invention is not limited to the embodiment described above, and that various modifications and changes may be made as will be described in modification examples below, and the modifications and changes are also encompassed in the technical scope of the present invention. Furthermore, the effects described in the above embodiment are merely the most preferred effects exerted by the present invention, and the effects of the present invention are not limited to those described in the embodiment. Although the embodiment described above and the modification examples described below can be appropriately combined for, detailed description thereof will be omitted.

### (Modification Examples)

In the locking stent 2, it is sufficient that at least one cell 20a having the open cell portions 21 and 22 be provided to the main body 11, in which, for example, other cells 20 may be cells each only have the open cell portion 21 or 22 or may be cells each have no open cell portions 21 and 22.

In the open cell portion 21 of the cell 20a, the other ends of the struts 24a and 24b may be each coupled to another strut in the cell arrangement direction SD. According to the present configuration, although the two struts do not extend in the major axis direction LD when the locking stent 2 is set in the reduced diameter state, the vertex 25 protrudes in the circumferential direction RD when the locking stent 2 is set in the natural state (and the expanded diameter state). Accordingly, it is possible to expect an improvement in anchoring force in the expanded diameter state.

In the open cell portion 27 of the cell 20a, the other ends of the two struts 24h and 24i are each coupled to a strut of another adjacent cell 20 in the cell arrangement direction SD or the closed cell portion 23 (see FIG. 5). In the present configuration, since the shape of the open cell portion 27 is formed into a substantially L-shape, and the angle of the vertex is appropriately set, it is possible to allow the two struts to extend in the major axis direction LD when the locking stent 2 is set in the reduced diameter state.

In the locking stent 2, the mesh-patterned structure in which the cells 20 are arranged in the major axis direction is not limited to the structure in which, as illustrated in the embodiment, the cells 20 are arranged helically with respect to the major axis direction LD. For example, the mesh-patterned structure may include one in which the cells 20 are arranged in not only the major axis direction LD but also the circumferential direction RD, or another in which the cells 20 are arranged only in the major axis direction LD. That is, it is sufficient that the stent 2 have a mesh-patterned structure in which the cells 20 are arranged at least in the major axis direction LD.

The locking stent 2 has been used in an application in which the locking stent is made to temporarily indwell in a blood vessel and then collected out of a body. The locking stent 2 may also be used in another application in which the locking stent 2 is not collected from inside the body, but is left permanently in the blood vessel. In a case of use in such an application, for example, such a configuration may be applied in which a coupling part between the locking stent 2 and the delivery wire 3 includes a heater coil or filament, for example, and electric power is supplied to the delivery wire 3 to allow the coupling part to be cut (fusion-cut). Furthermore, such a configuration may also be applied in which the coupling part between the locking stent 2 and the delivery wire 3 is mechanically cut when a rotational force is applied in a circumferential direction to the delivery wire 3. The coupling part between the locking stent 2 and the delivery wire 3 is not limited to have one of the configurations in the examples described above, and any desired configuration may also be applied as long as it is possible to separate the locking stent 2 and the delivery wire 3 from each other through an operation by a practitioner.

### EXPLANATION OF REFERENCE NUMERALS

1: Distal stabilizer
2: Locking stent
3: Delivery wire
4: Indwelling stent
5: First catheter
6: Second catheter
10: Delivery system
11: Main body
12: Antenna portion
20, 20a, 20b: Cell
21 (21a, 21b), 22, 27: Open cell portion
23: Closed cell portion
24 (24a - 24i): Strut
25, 26, 28: Vertex

## Claims

1. A distal stabilizer designed for catheter delivery in a biological lumen, the distal stabilizer comprising:
a linear delivery member; and
a cylindrical part coupled to the linear delivery member and locked to an inner wall of the biological lumen,
wherein
the cylindrical part includes a main body having a mesh-patterned structure formed of cells, each enclosed by wire-shaped members, the cells arranged in at least a major axis direction,
at least one cell among the cells forming the main body has at least one open cell portion in which two wire-shaped members are coupled to each other at a vertex, and the at least one open cell portion includes the two wire-shaped members that extend in the major axis direction in a reduced diameter state and the vertex that protrudes in a circumferential direction orthogonal to the major axis direction in a natural state.

2. The distal stabilizer according to claim 1, wherein the open cell portion includes the vertex that protrudes in a direction at angles ranging from -30° to +30° inclusive with respect to the circumferential direction of the cylindrical part in the natural state.

3. The distal stabilizer according to claim 1 or 2, wherein the open cell portion is provided to at least one cell among the cells, the at least one cell being positioned at a distal end in the major axis direction of the main body.

4. The distal stabilizer according to claim 1 or 2, wherein the open cell portion is provided to at least one cell among the cells, the at least one cell being positioned at a proximal end in the major axis direction of the main body.

5. The distal stabilizer according to claim 1 or 2, wherein the open cell portion is provided to at least one cell among the cells, the at least one cell being positioned between a distal end and a proximal end in the major axis direction of the main body.

6. The distal stabilizer according to claim 1 or 2, wherein the open cell portion is also provided to an antenna portion coupling a proximal end of the main body to the linear delivery member.

7. The distal stabilizer according to claim 1 or 2, wherein at least one cell among the cells, which has the open cell portion, includes an open cell portion in which some of the wire-shaped members do not extend substantially in the major axis direction in the reduced diameter state, but protrude in the circumferential direction orthogonal to the major axis direction in the natural state.

8. The distal stabilizer according to claim 1 or 2, wherein at least one cell among the cells, which has the open cell portion, includes an open cell portion in which some of the wire-shaped members do not extend substantially in the major axis direction in the reduced diameter state and the natural state.

9. The distal stabilizer according to claim 1 or 2, wherein the cylindrical part has a structure in which the cells are arranged helically with respect to the major axis direction.

10. The distal stabilizer according to claim 1 or 2, wherein the distal stabilizer is designed for an application in which the distal stabilizer is made to temporarily indwell in the biological lumen and then collected out of a body.
